# EUROPÄISCHE PATENTSCHRIFT

(11) **EP 1 038 034 B1**
(45) Veröffentlichungstag und Bekanntmachung des Hinweises auf die Patenterteilung: **27.10.2004**
(21) Anmeldenummer: 98965768.9
(22) Anmeldetag: 04.12.1998
(51) Int. Cl.: C12Q 1/68

(54) **VERFAHREN ZUR IDENTIFIKATION VON NUCLEINSÄUREN DURCH ELEKTROSPRAY MASSENSPEKTROMETRIE**
METHOD FOR IDENTIFYING NUCLEIC ACIDS BY ELECTRO-SPRAY MASS SPECTROMETRY
PROCEDE D'IDENTIFICATION D'ACIDES NUCLEIQUES PAR SPECTROMETRIE DE MASSE PAR ELECTROPULVERISATION

(30) Priorität: 05.12.1997 EP 97121470; 12.12.1997 EP 97121983
(43) Veröffentlichungstag der Anmeldung: 27.09.2000
(73) Patentinhaber: MAX-PLANCK-GESELLSCHAFT ZUR FÖRDERUNG DER WISSENSCHAFTEN E.V., 14195 Berlin (DE)
(72) Erfinder: GUT, Ivo, Glynne, 75014 Paris (FR); BERLIN, Kurt, D-14532 Stahnsdorf (DE); LEHRACH, Hans, D-14195 Berlin (DE)
(74) Vertreter: Wachenfeld, Joachim, Dr.
(86) Internationale Anmeldenummer: PCT/EP1998/007909
(87) Internationale Veröffentlichungsnummer: WO 1999/029897

(56) Entgegenhaltungen:
- EP-A- 0 303 459
- US-A- 5 635 404
- ARLINGHAUS H F ET AL: "MULTIPLEXED DNA SEQUENCING AND DIAGNOSTICS BY HYBRIDIZATION WITH ENRICHED STABLE ISOTOPE LABELS" ANALYTICAL CHEMISTRY, Bd. 69, Nr. 8, 15. April 1997, Seiten 1510-1517, XP000690164 in der Anmeldung erwähnt
- LITTLE D P ET AL: "RAPID SEQUENCING OF OLIGONUCLEOTIDES BY HIGH-RESOLUTION MASS SPECTROMETRY" JOURNAL OF THE AMERICAN CHEMICAL SOCIETY, Bd. 116, 1994, Seiten 4893-4897, XP002072824
- REDDY ET AL.: "Analysis of synthetic oligodeoxynucleotides containing modified components by electrospray ionization mass spectrometry" ANALYTICAL CHEMISTRY, Bd. 220, 1994, Seiten 200-207, XP002099463 in der Anmeldung erwähnt

## Beschreibung

Die vorliegende Erfindung betrifft ein Verfahren zum Nachweis einer Nucleotidsequenz in einem Nucleinsäuremolekül vermittels vorbestimmter Sonden unterschiedlicher Masse durch Elektrospray Massenspektrometrie. Vorteilhafterweise erlaubt das erfindungsgemäße Verfahren die gleichzeitige Charakterisierung einer Vielzahl unbekannter Nucleinsäuremoleküle durch einen Satz verschiedener Sonden.

Die genaue Charakterisierung von Nucleinsäuren ist sehr aufwendig und teuer. Unbekannte DNA kann charakterisiert werden, indem sie sequenziert wird. Dies ist die präziseste Art, DNA zu analysieren. Jedoch ist die Sequenzierung von DNA sehr aufwendig und nur dann notwendig, wenn die gesamte Sequenz von Interesse ist. Nur sehr kurze DNA-Ausschnitte (<1000 Nucleobasen) können in einem Arbeitsgang sequenziert werden. Wenn DNA Fragmente, die größer als diese 1000 Nucleobasen lang sind, in größerem Umfang analysiert werden sollen, ist es notwendig die DNA zu unterteilen, was die Methode verteuert.

Viele Aussagen lassen sich jedoch bereits mit geringerer Auflösung machen. Die derzeit beschriebenen Verfahren leiden jedoch darunter, daß unter Umständen Radioaktivität verwendet werden muß, und nur eine einzige Sonde in einer Analyse verwendet werden kann. Ein solches Verfahren aus dem Stand der Technik beinhaltet zum Beispiel, partielle Information über einen Array von verschiedenen Ziel-DNAs zu suchen. Ein Array mit vielen tausend Ziel-DNAs kann auf einer Festphase immobilisiert und anschließend können alle Ziel-DNAs gemeinsam auf das Vorhandensein einer Sequenz mittels einer Sonde (Nucleinsäure mit komplementärer Sequenz) untersucht werden.^{1,2} Eine Übereinstimmung in der Ziel-DNA mit der Sonde kann durch eine Hybridisation der beiden Nucleinsäuren nachgewiesen werden. Sonden können beliebige Nucleinsäuresequenzen unterschiedlicher Länge sein. Es existieren verschiedene Verfahren für die Auswahl von optimalen Bibliotheken von Sondensequenzen, welche minimal miteinander überlappen.^{3, 4} Sondensequenzen können auch gezielt zusammengestellt werden, um bestimmte Ziel-DNA Sequenzen aufzufinden. Oligofingerprinting ist ein Ansatz, bei welchem diese Technologie zum Einsatz kommt. Eine Bibliothek von Ziel-DNAs wird mit kurzen Nucleinsäuresonden abgetastet. Meist sind hier die Sonden nur 8-12 Basen lang. Es wird jeweils eine Sonde auf einmal an eine auf einer Nylonmembran immobilisierte Ziel-DNA-Bibliothek hybridisiert. Die Sonde ist radioaktiv markiert und die Hybridisierung wird anhand der Lokalisierung der Radioaktivität beurteilt. Für die Abtastung eines immobilisierten DNA-Arrays sind auch bereits fluoreszent markierte Sonden verwendet worden.⁵ Ein ähnliches Verfahren wird verwendet, um Sequenzierung von DNA zu multiplexen.^{6.7} Verschiedene Vektorsysteme werden zur Klonierung von Zielsequenzen eingesetzt. Jeweils ein Klon von jedem Klonierungsvektor wird gepoolt, die Sequenzierungsreaktion durchgeführt, die Fragmente auf einem Gel aufgetrennt und das Gel auf eine Nylonmembran geblottet. An die immobilisierte DNA werden anschließend die unterschiedlichen Sequenzen des Klonierungssystems hybridisiert, so daß man die zum jeweiligen Klonierungssystem gehörende Sequenz erhält. Hier kann die Abtastung des Klonierungssystems auch mittels einer massenspektrometrisch detektierbaren Sonde erfolgen.⁸

Als Sonden kommen jegliche Moleküle in Frage, die sequenzspezifisch mit einer Ziel-DNA wechselwirken können. Am gängigsten sind Oligodeoxyribonucleotide. Für diesen Zweck bietet sich jedoch jede Modifikation von Nucleinsäuren an, z.B Peptide Nucleic Acids (PNA),^{9.10} Phosphorothioatoligonucleotide oder Methylphosphonatoligonucleotide. Die Spezifität einer Sonde ist sehr wesentlich. Phosphorothioatoligonucleotide sind nicht besonders bevorzugt, da ihre Struktur durch die Schwefelatome verändert wird, was einen negativen Einfluß auf die Hybridisierungseigenschaften hat. Dies kann daher stammen, daß Phosphorothioatoligonucleotide normalerweise nicht diastereomerenrein synthetisiert werden. Bei Methylphosphonatoligonucleotiden bestand in der Vergangenheit ein ähnliches Reinheitsproblem, jedoch werden diese Oligonucleotide vermehrt diastereomerenrein synthetisiert. Ein wesentlicher Unterschied zwischen Methylphosphonatoligonucleotiden und Phosphorothioatoligonucleotiden ist das bei ersteren ungeladene Rückgrat, welches zu einer verminderten Abhängigkeit der Hybridisation von Puffersalzen und insgesamt durch die geringere Abstoßung zu höherer Affinität führt. Peptide Nucleic Acids haben ebenfalls ein ungeladenes Rückgrat, welches gleichzeitig chemisch sehr stark von der gängigen Zucker-Phosphat Struktur des Rückgrats in Nucleinsäuren abweicht. Das Rückgrat einer PNA weist eine Amidsequenz anstelle des Zucker-Phosphat Rückgrats gewöhnlicher DNA auf. PNA hybridisiert sehr gut mit DNA komplementärer Sequenz. Die Schmelztemperatur eines PNA/DNA-Hybrids ist höher als die des entsprechenden DNA/DNA-Hybrids und wiederum ist die Abhängigkeit der Hybridisierung von Puffersalzen relativ gering.

Electrospray Massenspektrometrie (ESI) ist eine neue, sehr leistungsfähige Methode für die Analyse von Biomolekülen (Meng, C.K., Mann, M. und Fenn, J.B., Z. Phys. D. 1988, 10, 361-368; Siuzdak, G. Proc. Natl. Acad. Sci. USA, 1994, 91,11290-11297). Die grundsätzlichen Charakteristika der Methode sind, daß eine Lösung eines Gemisches von Biomolekülen durch eine metallische Nadel, an die eine hohe Spannung angelegt ist (typischerweise einige Kilovolt), ins Vakuum eines Massenspektrometers geleitet wird. Durch die elektrische Instabilität und die Druckverminderung dispergiert die Flüssigkeit in kleinen Tröpfchen, die jeweils auf ihrer Oberfläche eine elektrische Ladung mittragen. Über mehrere Skimmerstufen werden die Tröpfchen ins Hochvakuum des Massenspektrometers überführt. Dabei geht das Lösungsmittel vom Tröpfchen allmählich verloren und die nackten, mehrfach geladenen Biomoleküle befinden sich unfragmentiert im Hochvakuum des Massenspektrometers. Bei diesem Verfahren ergeben sich für den Schritt, der die massenspektrometrische Analyse umfaßt, mehrere Möglichkeiten. Eine davon betrifft die Auftrennung der Moleküle unterschiedlicher Masse und Ladung durch einen Magneten (Sektor oder Quadrupol); eine weitere betrifft die Analyse mittels der Flugzeit. Hierbei wird senkrecht zum Ionenstrahl eine Beschleunigungsspannung gepulst und die Teilchengröße anhand ihrer Flugzeit bestimmt. Mit diesen beiden Ausführungsformen läßt sich im Massenbereich bis mehrere Tausend Dalton Isotopenauflösung erzielen. Mit neuen Nanosprayquellen lassen sich mit wenigen 100 fmol Material ausgezeichnete Spektren aufzeichnen.
Bisher hat sich ESI im wesentlichen für die Analyse von Peptiden durchgesetzt (Siuzdak, G., Proc. Natl. Acad. Sci. USA, 1994, 91, 11290-11297) und sich insbesondere für die Analyse von kombinatorisch hergestellten Peptidbibliotheken bewährt (Metzger, J.W., Kempter, C., Wiesmuller, K.H. und Jung, G., Anal. Biochem., 1994, 219, 261-277; Winger, B.E., und Campana, J.E., Rapid Commun. Mass Spectrom., 1996, 10, 1811-1813). Die Analyse von Nukleinsäuren und modifizierten Varianten von Nukleinsäuren ist derzeit noch nicht oft berücksichtigt worden (Reddy, D.M., Rieger, R.A., Torres, M.C., und Iden C.R., Anal. Biochem. 1994, 220, 200-207; Potier, N., Van Dorsselaer, A., Cordier, Y., Roch, O. und Bischoff, R., Nucleic Acids Res., 1994, 22, 3895-3903; Greig, M. und Griffey, R.H., Rapid Commun. Mass Spectrom., 1995, 9, 97-102). Dabei stellten sich Probleme wie, daß Nukleinsäuremoleküle Polyanionen sind und dadurch zusätzliche Probleme in der Ionisation auftreten.

Kombinatorische Synthesen,¹³ d.h. die Herstellung von Substanzbibliotheken ausgehend von einem Gemisch von Vorstufen, werden sowohl auf fester als auch in flüssiger Phase durchgeführt. Vor allem die kombinatorische Festphasensynthese hat sich frühzeitig etabliert, da in diesem Fall die Abtrennung von Nebenprodukten besonders einfach ist. Nur die an den Träger (Support) gebundenen Zielverbindungen werden in einem Waschschritt zurückbehalten und am Ende der Synthese durch das gezielte Spalten eines Linkers isoliert. Diese Technik erlaubt auf einfachem Wege die gleichzeitige Synthese einer Vielzahl verschiedener Verbindungen an einer Festphase und somit den Erhalt von chemisch "reinen" Substanzbibliotheken.^{14,15,16} Daher sind die Verbindungsklassen, die auch in nicht kombinatorischen, konventionellen Synthesen auf einer Festphase synthetisiert werden, der kombinatorischen Chemie besonders leicht zugänglich und werden demzufolge auch breit verwendet. Dies trifft vor allem auf Peptid-, Nucleinsäure- und PNA-Bibliotheken zu.

Die Synthese von Peptiden erfolgt durch Binden der ersten N-geschützten Aminosäure (z.B. Boc) an den Support, nachfolgende Entschützung und Reaktion der zweiten Aminosäure mit der freigewordenen NH₂-Gruppe der ersten. Nicht reagierte Aminofunktionen werden in einem weiteren "Capping" Schritt einer Weiterreaktion im nächsten Synthesezyklus entzogen. Die Schutzgruppe an der Aminofunktion der zweiten Aminosäure wird entfernt und der nächste Baustein kann gekoppelt werden. Zur Synthese von Peptidbibliotheken wird ein Gemisch von Aminosäuren in einem oder mehreren Schritten verwendet. Die Synthese von PNA und PNA-Bibliotheken erfolgt sinngemäß.

Nucleinsäure-Bibliotheken werden meist durch Festphasensynthese mit Gemischen verschiedener Phosphoramidit-Nucleoside erhalten. Dies kann auf kommerziell erhältlichen DNA-Synthesizern ohne Veränderungen in den Syntheseprotokollen durchgeführt werden. Darüber hinaus sind verschiedene Arbeiten zur kombinatorischen Synthese von PNA Bibliotheken publiziert worden.^{17,18} Diese Arbeiten behandeln den Aufbau von kombinatorischen Sequenzen, d:h. die Synthese von PNAs in denen einzelne, spezifische Basen in der Sequenz durch degenerierte Basen ersetzt werden und dadurch zufällige Sequenzvarianz erreicht wird. Auch die Verwendung massenspektrometrischer Methoden für die Analyse kombinatorischer Bibliotheken ist mehrfach beschrieben worden.^{19,20,21,22}

Es existieren verschiedene Verfahren, um DNA zu immobilisieren. Das bekannteste Verfahren ist die Festbindung einer DNA, welche mit Biotin funktionalisiert ist, an eine Streptavidin-beschichtete Oberfläche.²³ Die Bindungsstärke dieses Systems entspricht einer kovalenten chemischen Bindung, ohne eine zu sein. Um eine Ziel-DNA kovalent an eine chemisch vorbereitete Oberfläche binden zu können, bedarf es einer entsprechenden Funktionalität der Ziel-DNA. DNA selbst besitzt keine Funktionalisierung, die dazu geeignet ist. Es gibt verschiedene Varianten, in eine Ziel-DNA eine geeignete Funktionalisierung einzuführen: Zwei leicht zu handhabende Funktionalisierungen sind primäre, aliphatische Amine und Thiole. Solche Amine werden quantitativ mit N-Hydroxy-succinimidestern umgesetzt. Thiole reagieren unter geeigneten Bedingungen quantitativ mit Alkyliodiden. Eine Schwierigkeit besteht im Einführen einer solchen Funktionalisierung in eine DNA. Die einfachste Variante ist die Einführung durch einen Primer einer PCR. Gezeigte Varianten benützen 5'-modifizierte Primer (NH₂ und SH) und einen bifunktionalen Linker.^{24,25,26}

Bei der Immobilisierung auf einer Oberfläche ist vor allem ihre Beschaffenheit von wesentlicher Bedeutung. Bis jetzt beschriebene Systeme sind hauptsächlich aus Silizium oder Metall (magnetic beads). Eine weitere Methode zur Bindung einer Ziel-DNA basiert darauf, eine kurze Erkennungssequenz (z.B. 20 Basen) in der Ziel-DNA zur Hybridisierung an ein oberflächenimmobilisiertes Oligonucleotid zu verwenden.²⁷ Es sind auch enzymatische Varianten zur Einführung von chemisch aktivierten Positionen in eine Ziel-DNA beschrieben worden.²⁸ Hier wird an einer Ziel-DNA enzymatisch eine 5'-NH₂-Funktionalisierung durchgeführt.

Wie vorstehend beschrieben, sind im Stand der Technik eine Reihe von Verfahren bekannt, die vor allem auf eine genaue Analyse von Nucleinsäuren ausgerichtet sind. Diese Verfahren sind in der Regel entweder sehr arbeitsaufwendig und/oder kostenintensiv.

Das der vorliegenden Erfindung zugrundeliegende technische Problem war somit, ein schnelles und kosteneffizientes Verfahren zur Identifikation von Ziel-Nucleinsäuren bereitzustellen.

Dieses technische Problem wird durch die Bereitstellung der in den Patentansprüchen gekennzeichneten Ausführungsformen gelöst.

Somit betrifft die vorliegende Erfindung ein Verfahren zum Nachweis einer Nucleotidsequenz in einem Nucleinsäuremolekül, das die folgenden Schritte umfaßt:
(a) Hybridisierung von Nucleinsäuremolekülen mit einem Satz von Sonden unterschiedlicher Nucleobasensequenzen, wobei jede Sonde eine von allen anderen Sonden verschiedene Masse aufweist;
(b) Abtrennung der nicht hybridisierten Sonden;
(c) Ablösen der spezifisch hybridisierten Sonden in ein Lösungsmittel;
(d) Analyse der hybridisierten Sonden in der Lösung mittels Electrospray Massenspektrometrie; und
(e) Bestimmung der Nucleinsäuremoleküle durch die daran hybridisierten Sonden.

Vorzugsweise sind die Nucleinsäuremoleküle auf einem Träger (Support) angeheftet. Bevorzugt ist dann, daß die Positionen der Sonden auf dem Probenträger eine Zuordnung zu dem damit hybridisierenden Nucleinsäuremolekül erlaubt. Der Begriff "Probenträger", wie im Sinne der vorliegenden Erfindung verwendet, bezeichnet jegliche Form von Trägermaterial.

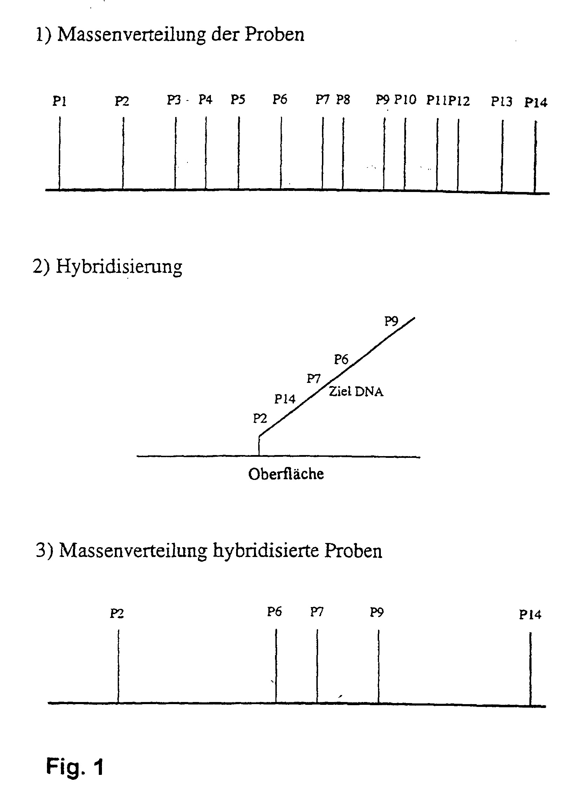

Das erfindungsgemäße Verfahren (vergleiche Figur 1) kombiniert in vorteilhafter Weise Verfahren, Ziel-Nucleinsäuren zu analysieren (Oligofingerprinting), sowie die massenspektrometrische Analyse von Nucleinsäuren und modifizierten Nucleinsäuren. Dabei wird eine Vielzahl von unterschiedlichen Sonden eingesetzt, die den Nachweis von einer oder mehreren Nucleotidsequenz(en) in einem Nucleinsäuremolekül erlauben. Eine Kombination dieser beiden Methoden konnte bisher nicht durchgeführt werden, weil die Massenunterscheidbarkeit der Sonden keine eindeutigen Rückschlüsse auf die Sequenz zuließ und die Empfindlichkeit der massenspektrometrischen Analyse von Nucleinsäuren nicht an die Probenmengen eines Oligofingerprinting-Experimentes angepaßt war. Durch die Hybridisierung einer bestimmten Sonde (oder mehrerer bestimmter Sonden) wird im weitesten Sinne der Erfindung ein die (im wesentlichen) komplementäre(n) Nucleotidsequenz(en) enthaltendes Nucleinsäuremolekül bestimmt. Durch diese Bestimmung wird auch der Nachweis geführt, daß eine bestimmte Nucleotidsequenz oder sehr ähnliche Sequenzen (z.B. bei Hybridisierung unter nicht stringenten Bedingungen, s.u.) in dem Molekül enthalten ist/sind. Dem Fachmann ist klar, daß eine gesuchte Nucleotidsequenz mit Hybridisierungsverfahren nicht immer absolut ermittelt werden kann, da auch unter stringenten Hybridisierungsbedingungen trotz sogenannter "Mismatches" unter Umständen eine Hybridisierung einer Sonde stattfinden kann (z.B. ab einer bestimmten Mindestlänge einer Sonde oder bei (einer) Positionierung(en) des/der Mismatches, die bei der Hybridisierung tolerierbar ist/sind). Bei einer vorgegebenen Nucleotidsequenz einer Sonde kann eine Komplementärsequenz im Nucleinsäuremolekül somit in einem Teil der Ausführungsformen nur mit einer gewissen Näherung ermittelt werden, da neben exakten Komplementärsequenzen unter Umständen auch solche ermittelt werden können, die in ihrer Sequenz nicht exakt komplementär sind. Insofern umfaßt die Nucleotidsequenz auch homologe Nucleotidsequenzen, die vorzugsweise einen Homologiegrad von mehr als 90 %, besonders bevorzugt von mehr als 95 % aufweisen. Alle diese vorstehenden Ausführungsformen sind von der vorliegenden Erfindung umfaßt.

Je nach Wahl der Hybridisierungsbedingungen kann das erfindungsgemäße Verfahren verwendet werden, um entweder spezifische Nucleotidsequenzen oder Gruppen von Nucleotidsequenzen nachzuweisen, die sich durch eine ähnliche Sequenz auszeichnen. Werden z.B. stringente Hybridisierungsbedingungen gewählt, so sind die verwendeten Sonden nur in der Lage, an die Nucleotidsequenzen zu hybridisieren, die exakt komplementär zu ihren Nucleobasensequenzen sind. Werden hingegen nicht stringente Hybridisierungsbedingungen gewählt, so kann man mit den eingesetzten Sonden alle Nucleotidsequenzen nachweisen, die von den Nucleobasensequenzen der Sonden derart abweichen, daß sie unter den gewählten Bedingungen eine Hybridisierung noch erlauben. Auf diese Art und Weise kann das erfindungsgemäße Verfahren also auch verwendet werden, um Homologe. Varianten oder Allele einer bestimmten Sequenz nachzuweisen. Dem Fachmann ist bekannt, was unter stringenten bzw. nicht stringenten Hybridisierungsbedingungen zu verstehen ist; vgl. z. B. Sambrook et al., "Molecular Cloning, A Laboratory Manual" CSH Press, Cold Spring Harbor, 2nd ed. 1989, Hames und Higgins (Hrsg.) "Nucleic Acid Hybridization, A Practical Approach", IRL Press, Oxford 1985. Stringente Hybridisierungsbedingungen sind, zum Beispiel, Hybridisierung in 6 x SSC, 5 x Denhardt's Reagens, 0,5 % SDS und 100 µg/ml denaturierter DNA bei 65 °C und Waschen in 0,1 x SSC, 0,1 % SDS bei 65 °C. Nicht-stringente Hybridisierungsbedingungen unterscheiden sich von den obengenannten Bedingungen insofern, als beispielsweise die Hybridisierung und/oder das Waschen bei einer niedrigeren Temperatur durchgeführt werden, z.B. bei 50 °C oder 55 °C und/oder die Menge von SSC auf z.B. 1 x oder 2 x SSC angehoben wird.

Das erfindungsgemäße Verfahren erlaubt auch den Nachweis mehrerer unterschiedlicher Sequenzen in einer Ziel-DNA, wobei die unterschiedlichen Sequenzen komplementär zu verschiedenen Sonden sind. Günstigstenfalls, z. B. bei Verwendung von Sonden mit überlappenden Sequenzen, läßt sich so die gesamte Nucleotidsequenz einer Ziel-Nucleinsäure nachweisen oder aufklären.

Mit dem erfindungsgemäßen Verfahren läßt sich zunächst bestimmen, ob eine Ziel-Nucleinsäure am Immobilisierungssupport angeheftet war, die eine mit einer Sonde unter den gewählten Bedingungen hybridisierbare Sequenz aufweist. Sofern dies der Fall ist, kann die Proben-Nucleinsäure weiter analysiert und charakterisiert werden. Da für das erfindungsgemäße Verfahren üblicherweise nur ein Bruchteil einer Probe für die erfindungsgemäße Analyse eingesetzt werden muß, kann die nicht eingesetzte Nucleinsäure mit Standardverfahren. z. B. Sequenzierungsverfahren, weiter untersucht werden.

Die Ablösung der spezifisch hybridisierten Sonden in ein Lösungsmittel erfolgt vorzugsweise durch Hitzedenaturierung oder alkalische Denaturierung. Als Lösungsmittel eignen sich insbesonders wäßrige Lösungen oder Gemische von Wasser und organischen Lösungsmitteln, sofern das organische Lösungsmittel mit Wasser mischbar ist (z.B. Wasser und Acetonitril). Vorzugsweise werden die Gemische im Verhältnis 1:1 eingesetzt. Da eine Denaturierung der hybridisierten Moleküle erzielt werden soll, ist die Wahl des Gemisches zudem von der Art der hybridisierten Moleküle abhängig.

Das erfindungsgemäße Verfahren kann auch, parallel oder sukzessive, mehrmals durchgeführt werden, wobei die Hybridisierungsbedingungen variiert werden. So kann man beispielsweise in einer Ziel-DNA zunächst feststellen, ob sie einen bestimmten Homologiegrad mit anderen Ziel-DNAs aufweist, bevor nach spezifischen Sequenzen gesucht wird.

Die Zuordnung der an einer bestimmten Position auf dem Probenträger hybridisierten Sonde zur immobilisierten Probe erfolgt vorzugsweise mittels eines Datenverarbeitungssystems, das das jeweilige aufgenommene Spektrum auf einer Position des Probenträgers einer auf derselben Position angebrachten Ziel-DNA zuordnet. Vorzugsweise sind die Ziel-Nucleinsäuren auf der Oberfläche bzw. auf dem Proteinträger in einer bestimmten Ordnung angeordnet.

In einer bevorzugten Ausführungsform des erfindungsgemäßen Verfahrens werden die Nucleinsäuremoleküle vor oder nach Schritt (a) auf die Oberfläche eines Trägers übertragen. Dazu eignet sich z.B. ein "magnetic bead", welches so funktionalisiert ist, daß sich eine entsprechend funktionalisierte Ziel-DNA daran binden läßt, oder eine Funktionalisierung z.B. einer Mikrotiterplatte, in deren verschiedenen Vertiefungen verschiedene Ziel-DNAs immoblisiert werden können. So lassen sich Hybridisierungsschritte mit Sondenbibliotheken und nachfolgende Waschschritte vereinfachen. Als Immobilisierungssysteme bieten sich z.B. Biotin-Streptavidin, NH₂-N-Hydroxysuccinimidylester oder SHalkylhalogenid an.

In einer weiteren bevorzugten Ausführungsform des erfindungsgemäßen Verfahrens erfolgt die Immobilisierung der Nucleinsäuremoleküle an der Probenträgeroberfläche über eine NH₂-, Epoxy- oder SH-Funktion, vermittels der Beschichtung der Probenträgeroberfläche mit einem Silikat oder Silan, über eine Protein-Substrat-, Protein-Protein- oder eine Protein-Nucleinsäure-Interaktion, oder über eine Wechselwirkung zweier hydrophober Komponenten.

Wird der Probenträger z.B. mit Gold beschichtet, kann eine Kopplung der Ziel-DNA durch während der molekularbiologischen Aufbereitung der Ziel-DNA eingeführte SH- oder NH₂-Funktionen erfolgen. Auch die umgekehrte Möglichkeit, entsprechend modifizierte DNA an funktionalisierte Goldpartikel zu binden, besteht. Die Firma Nanoprobes Inc., Stony Brook, NY, bietet beispielsweise mit Streptavidin oder mit Aminofunktionen verknüpfte Gold-Nanopartikel kommerziell an. Durch eine Aminofunktionalisierung kann anschließend eine Kopplung der Ziel-DNA, welche über eine SH-Funktionalisierung an einen bifunktionalen Linker (z.B. SIAB, Pierce Chemical, Rockford, IL, USA) gebunden ist, an eine Glasoberfläche erreicht werden. Eine weitere Variante ist die unmittelbare Beschichtung der Oberfläche mit Trimethoxy-3-aminopropylsilan. An die Aminofunktion kann anschließend eine Ziel-DNA, wie oben über einen bifunktionalen Linker, gekoppelt werden.

In einer besonders bevorzugten Ausführungsform ist dabei die Protein-Substrat-Interaktion eine Biotin-Streptavidin- oder eine Antikörper-Antigen-Bindung. Viele Probenträger sind üblicherweise aus einem Material, an das sich ohne weitere Modifikation weder Proteine noch DNA-Moleküle immobilisieren lassen. Eine Möglichkeit der Immobilisierung ist, die Oberfläche mit Gold zu beschichten, da sich z. B. SH-Funktionen daran binden lassen. Für die Kopplung bieten sich dann bifunktionale Linker an, die eine SH-Funktion und eine weitere Funktion besitzen, die an die Funktionalisierung der Ziel-DNA angepaßt ist. Ist z. B. die Ziel-DNA biotinfunktionalisiert, sollte der Linker mit Streptavidin gekoppelt werden können. Ist die Ziel-DNA NH₂-funktionalisiert, dann kann der Linker mit einer N-hydroxysuccinimidylester-Funktion versehen sein.

In einer weiteren besonders bevorzugten Ausführungsform ist die Protein-Nucleinsäure-Interaktion eine Bindung der Nucleinsäure an Gene32, ein Protein, welches sequenzunspezifisch einzelsträngige DNA bindet.

In einer ebenfalls bevorzugten Ausführungsform des erfindungsgemäßen Verfahrens sind die verwendeten Sonden Nucleinsäuren, die mit einem Massen-Tag versehen sind. Gemäß dieser Ausführungsform können die Sonden auch mit mehreren Tags versehen sein, die an unterschiedlichen Positionen, z. B. am 5" und 3' Ende lokalisiert sind. Durch Kombination der Anzahl und Lokalisierung von Massen-Tags, gegebenenfalls in Kombination mit Ladungs-Tags ("Charge-Tags"), läßt sich die Versalität und Empfindlichkeit des erfindungsgemäßen Verfahrens deutlich erhöhen.

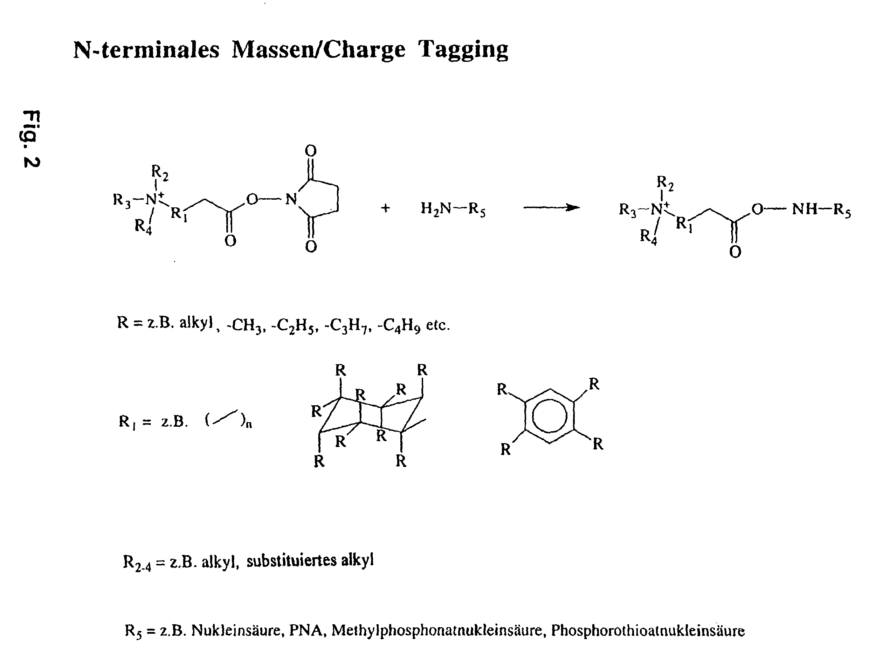

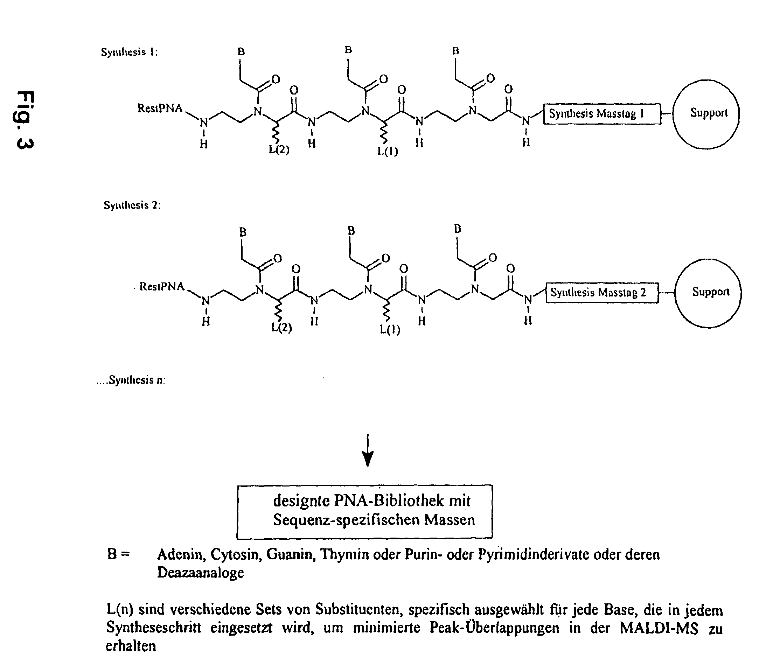

In einer besonders bevorzugten Ausführungsform sind die Massen-Tags zugleich Ladungs-Tags, während in einer weiteren besonders bevorzugten Ausführungsform die Nucleinsäuren zusätzlich mit einem Ladungs-Tag ("Charge-Tags") versehen sind (vgl. Figur 2).

"Charge tagging" kann entsprechend der Methode von Gut et al. durchgeführt werden.^{11,12} Ein aminofunktionalisiertes Substrat (1 mM) wird in Trimethylamin/CO₂-Puffer (pH = 8.5, 200 mM) auf Eis bei 0 °C mit 1% ω-Trimethylammoniumhexansäure-N-hydroxy-succinimidylester (CT) versetzt. Nach 30 Minuten werden der flüchtige Puffer und das Lösungsmittel im Vakuum abgezogen. Das aminofunktionalisierte Substrat kann z.B. eine kombinatorisch hergestellte Bibliothek von massenverschiedenen Sonden sein. Durch die Variation der Länge und Funktionalisierung des CT lassen sich die Massen der Substratbibliothek um einen definierten Betrag verschieben (Fig. 2). Da in der kombinatorischen Synthese z.B. eine Sondenbibliothek von 64 massenverschiedenen Sonden, die sich über einen Massenbereich von 200 Da erstrecken, hergestellt werden, erhöhen die Massen/charge tags jeweils in Einheiten von 200 Da die Masse. d.h. der erste kombinatorische Syntheseansatz wird mit dem kleinstmöglichen charge tag hergestellt, der zweite mit einem Massen/charge (Ladungs-) tag, das 200 Da schwerer ist, der dritte mit einem Massen/charge tag, das weitere 200 Da schwerer ist usw. Theoretisch kann der Bereich beliebig ausgeweitet werden, solange das angewendete Massenspektrometer in der Lage ist, den Unterschied zwischen zwei massenbenachbarten Sonden aufzulösen und solange die Synthese präparativ praktikabel erscheint. Für Sonden mit 10 Nucleobasen bekommt man ein Grundmasse im Bereich von 2600-2800 Da. Mit derzeit käuflichen Massenspektrometern ist der benützbare Massenbereich mit ausreichender Massengenauigkeit unter 4000 Da. Dadurch können sieben Ensembles von 64 Sonden verwendet werden (insgesamt 448 Sonden).

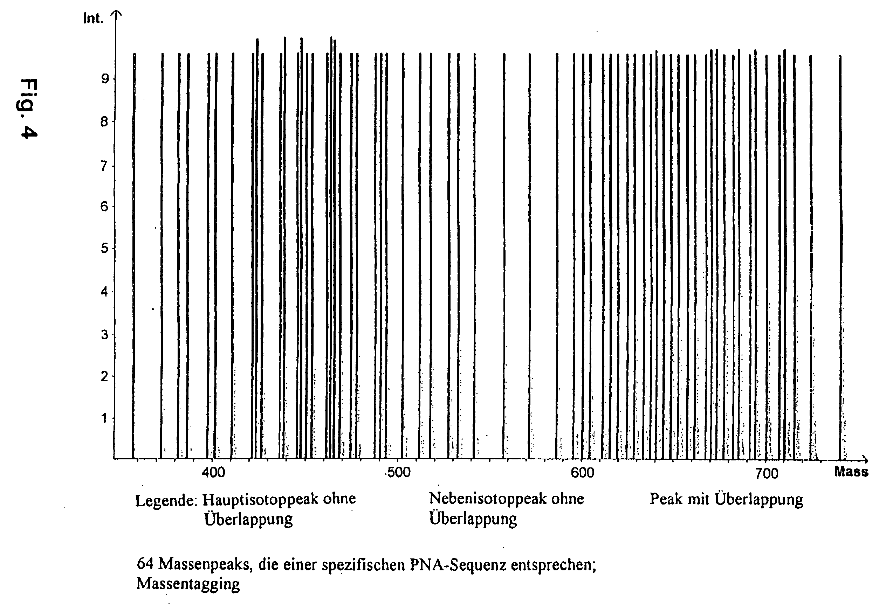

Die Synthese von Peptiden auf einem automatisierten Synthesizer verläuft vom C-terminalen Ende zum N-terminalen Ende, die Synthese von Nucleinsäuren vom 3'- zum 5'-Ende. Es besteht die Möglichkeit, an einem oder an beiden Enden eine primäre Aminofunktion anzubringen, um dann durch eine oder zwei Funktionalisierungen die Massenverschiebung zu erreichen. Alternativ kann die Massenverschiebung einer Bibliothek von kombinatorisch hergestellten Sonden auch erreicht werden, indem vorgängig zum Einbau der kombinatorischen Bausteine einige Bausteine von definierter Masse (z.B. Aminosäuren in eine kombinatorische Synthese von PNAs) angebracht werden. Die erste kombinatorische Synthese beginnt unmittelbar am Support. Für die zweite kombinatorische Synthese werden zuerst z.B. zwei Valine gekoppelt. Valin hat eine Masse von 99 Da. Durch zwei Valine erreicht man eine Massenverschiebung der zweiten kombinatorischen Synthese von der ersten um 198 Da. Bei der dritten kombinatorischen Synthese werden zuerst vier Valine gekoppelt, wodurch die Masse dieses Ensembles 396 Da höher ist usw. Ein allfällig nötiges charge tag kann immer noch nachträglich mit der oben beschriebenen Methode am N-terminalen Ende angebracht werden. Eine weitere Möglichkeit besteht darin, die charge tags zuerst an die Festphase zu koppeln und anschließend mit der kombinatorischen Synthese fortzufahren (vgl. z.B. Fig. 3).

Eine weitere Variante besteht darin, gleichzeitig mehrere fixe Ladungen der gleichen Polarität anzubringen. Dadurch kann der Detektionsbereich vermindert werden. In einem Massenspektrometer wird ein Molekül mit zwei Ladungen bei der entsprechend halben Masse beobachtet. Dies kann einen Vorteil bringen, wenn die Auflösung des Massenspektrometers zu höheren Massen hin stark abnimmt. Vorstehend ist beschrieben, wie man positive fixe Ladungen an einer Sonde festbringen kann. Negative fixe Ladungen können ähnliche Vorteile bringen. Positive und negative fixe Ladungen lassen sich mit ähnlichen Methoden herstellen.

Für die Lokalisierung, Anzahl und Kombinatorik der Ladungs-Tags gelten die im Zusammenhang mit den Massen-Tags gemachten Ausführungen entsprechend. So kann mittels charge tagging am 5'- oder 3'- Terminus der Bibliothek von Nucleinsäure-Sonden die Empfindlichkeit der Analyse verbessert werden. Dies gilt insbesondere, wenn durch die Einführung von Alkylphosphonatgruppen an den nicht randomisierten Positionen (vgl. unten) ansonsten Ladungsneutralität hergestellt wird. Durch das mit dem Charge Tagging zwangsläufig verbundene Massen Tagging wird zusätzlich die Analyse der Fragmente aus der Spaltung an den Phosphorothioatgruppen erleichtert. Phosphoroselenoate können ebenfalls eingesetzt werden, um eine bevorzugte Spaltung an bestimmten Positionen zu erhalten.

In einer weiteren bevorzugten Ausführungsform des erfindungsgemäßen Verfahrens sind die Sonden modifizierte Nucleinsäuremoleküle.

In einer besonders bevorzugten Ausführungsform handelt es sich bei diesen modifizierten Nucleinsäuremolekülen um PNAs, alkylierte Phosphorothioatnucleinsäuren oder Alkylphosphonatnucleinsäuren.

In einer weiteren bevorzugten Ausführungsform werden die Sonden des erfindungsgemäßen Verfahrens durch kombinatorische Festphasensynthese hergestellt. Das erfolgt über z.B. über die übliche Boc-Festphasensynthese, die auch zur Synthese von PNA-Reinsubstanzen kommerziell angewandt wird. Dabei werden in ausgewählten Positionen innerhalb einer sonst festgelegten Sequenz der Sonden-PNA alle vier, oder mehr als eine der Basen Adenin, Guanin, Cytosin oder Thymin nebeneinander eingebaut. Dies erfolgt durch den Einsatz nicht nur eines, sondern mehrerer Synthesebausteine in einem Schritt in der Festphasensynthese. Durch Variationen in mehreren solchen Positionen entsteht eine PNA-Bibliothek. Im Anschluß an die PNA-Synthese kann ein charge tagging an der freien terminalen Aminofunktion durchgeführt werden. Dies verbessert den dynamischen Bereich der PNA-Analyse mittels ESI.

In einer besonders bevorzugten Ausführungsform werden deshalb bei der Festphasensynthese die unterschiedlichen Basenbausteine derart markiert, daß ihre Massen bzw. die Massen der aus ihnen synthetisierten Sonden im Massenspektrometer unterscheidbar sind. Diese Markierung wird so bewerkstelligt, daß zur Base korrespondierend am Rückrat eine unterschiedliche Massenmodifikation eingeführt wird. Auf diese Art und Weise erhalten die synthetisierten Sonden Massen, die spezifisch für ihre Sequenzen sind. Binden nun die Sonden spezifisch an eine immobilisierte Ziel-DNA, so erlauben die im massenspektrometrischen Experiment zugänglichen Masseninformationen eindeutige Rückschlüsse auf die Sequenzen der jeweils hybridisierten Sonden.

In einer weiteren besonders bevorzugten Ausführungsform des erfindungsgemäßen Verfahrens werden die Basenbausteine mit einer Methyl-, Ethyl-, Propyl-, einer verzweigten oder unverzweigten Alkyl-, einer halogensubstituierten verzweigten oder unverzweigten Alkyl-, oder Alkoxyalkyl-, Alkylaryl-, Arylalkyl-, Alkoxyaryl- oder Aryloxyalkyl-Gruppe oder mit einer deren deuterierten oder sonstigen isotopen Varianten markiert. Da bei Verwendung nicht massenmodifizierter Bausteine die Molekülmasse allenfalls Rückschlüsse auf die Basenzusammensetzung, nicht aber auf die Sequenz erlaubt, werden massengelabelte Bausteine (d.h. am Rückgrat substituierte PNA Monomere) verwendet, die für jede randomisierte Position innerhalb der PNA-Bibliothek charakteristisch sind. Da auf diese Weise der Synthesebaustein einer bestimmten Base in der Position x eine andere Masse hat als in der Position y, werden auch unterschiedliche Sequenzen bei gleicher Bruttozusammensetzung der Basen über die Molekülmasse unterscheidbar. Die jeweiligen Substituenten werden durch numerische Rechnung so ausgewählt, daß jeder denkbaren Sequenz aufgrund der beschriebenen Art der Bibliothekssynthese eine festgelegte Masse eindeutig entspricht.

Die Synthese von Nucleinsäure-Sonden-Bibliotheken erfolgt am Synthesizer, indem an den zu randomisierenden Positionen Gemische verschiedener Nucleosidderivate (in der Regel Phosphoramidite) eingesetzt werden. Die sich daraus ergebenden Bibliotheken können ebenfalls als Sonden im oben beschriebenen Verfahren eingesetzt werden. Zur Unterscheidung der verschiedenen Sequenzen innerhalb der Bibliothek soll ein gezielter Bindungsbruch z.B. an festgelegten Phosphodiesterbindungen durchgeführt werden, die sich auf einer festgelegten Seite der randomisierten Positionen befinden.

In einer weiteren bevorzugten Ausführungsform des erfindungsgemäßen Verfahrens sind somit die Sonden mit mindestens einer Modifikation in einer definierten Position von randomisierten Nucleotiden entfernt versehen, die eine Spaltung der Sonde erlauben.

In einer besonders bevorzugten Ausführungsform handelt es sich bei dieser Modifikation um die Einführung einer Phosphorothioatgruppe und/oder einer RNA-Base und/oder einer Phosphotriesterbindung.

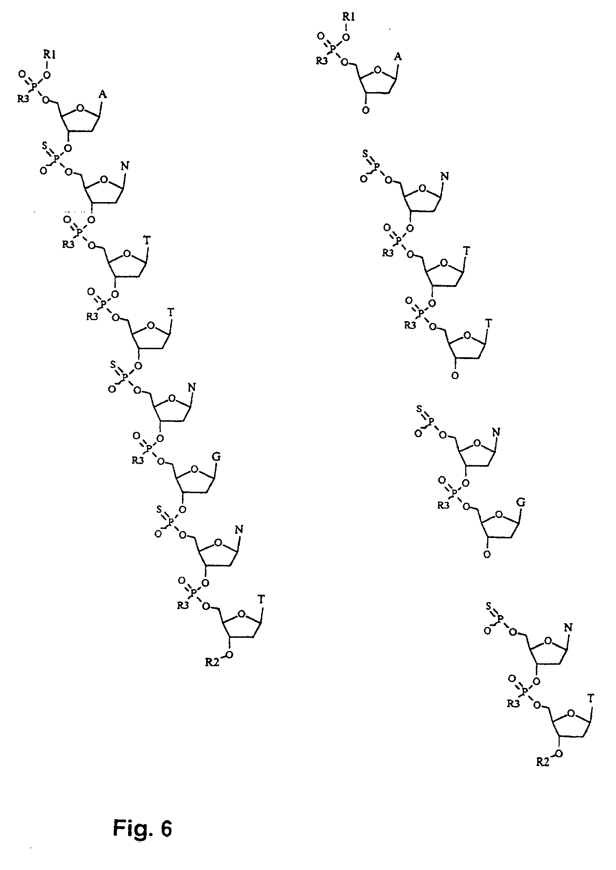

Enthält eine Sonde drei randomisierte Positionen, so sind mindestens zwei solcher Bindungsbrüche erforderlich (Fig. 6). Es ergeben sich dann drei Fragmente, die jeweils eine randomisierte Position enthalten und damit aufgrund der sonst bekannten Zusammensetzung über ihre Masse unmittelbar auf die variable Base schließen lassen. Die Spaltung an den beschriebenen Bindungen kann unvollständig erfolgen. Dies erlaubt die Einbeziehung größerer Fragmente, um die Sequenzinformation abzusichern oder bei Mehrdeutigkeiten zu konkretisieren. Der spezifische Bindungsbruch an den randomisierten Positionen wird durchgeführt, indem bereits bei der Synthese der Bibliothek dort Phosphorothioatgruppen eingeführt werden. Diese können mit Hydroxyalkylhalogeniden zuerst hydroxyalkyliert und dann unter basischen Bedingungen selektiv gespalten werden. Alternativ können Proben so aufgebaut werden, daß neben einer randomisierten Position Uracil eingebaut wird. Mittels Uracil-DNA Glycosylase und nachfolgender alkalischer Behandlung kann das Rückgrat dann an dieser Stelle gebrochen werden.

Das oben beschriebene Prinzip des Massentaggings für bestimmte Bausteine in festgelegten Positionen kann für verschiedene Teilbibliotheken angewendet werden, die dann wiederum zu einer größeren Bibliothek vereinigt werden können.

In einer weiteren bevorzugten Ausführungsform werden deshalb die Sonden als Teilbibliotheken hergestellt, die mit unterschiedlichen Massen- und/oder Ladungs-Tags versehen sind. Um von einer bestimmten analysierten Masse auf eine bestimmte Teilbibliothek rückschließen zu können, müssen auch diese Teilbibliotheken in ihrer Synthese massengelabelt werden. Dies erfolgt zweckmäßig über natürliche Aminosäuren, die sich leicht in einer Festphasensynthese an die PNA-Bibliothek binden lassen. Durch das Massenlabeln verschiedener Teilbibliotheken läßt sich der Massenbereich, der für die Analyse der Gesamtbibliothek zur Verfügung steht, beträchtlich aufweiten.

Weiterhin wird Kit offenbart, der die Sonden und/oder Teile eines Immobilisierungssystems (Probenträger), gegebenenfalls mit den gebundenen Nucleinsäuremolekülen, enthält. Als Immobilisierungssystem eignen sich z.B. (beschichtete) "magnetic beads" oder eine (beschichtete) Mikrotiterplatte. Wie vorstehend dargelegt, ist die Oberfläche des Immobilisierungssystems vorbehandelt und erlaubt somit die Bindung der Nucleinsäuren. Vorzugsweise erfolgt die Vorbehandlung auf chemischem Wege.

Die Figuren zeigen:
Figur 1
   Schema des Fingerprintings mit massenspektrometrischer Abtastung. 1) Kombinatorisch hergestellte Bibliothek von massenunterscheidbaren Sonden. 2) Die Bibliothek wird an eine Ziel-DNA, die immobilisiert ist, hybridisiert. Nach intensivem Waschen (um unspezifisch gebundene Sonden abzuwaschen) werden die korrekt hybridisierten Sonden von der Ziel-DNA abgelöst. 3) Diese Lösung wird in einem Electrospray Massenspektrometer analysiert. Durch die eindeutige Zuordnung von Massen zu Sequenzen werden Teilsequenzen der Ziel-DNA bestimmt.
Figur 2
   N-terminales Massen/charge Tagging.
   Am 5' einer Nucleinsäure oder einer modifizierten Version einer Nucleinsäure oder am N-terminalen Ende einer PNA kann ein charge tag eingeführt werden.
   Das charge tag trägt eine N-Hydroxy-succinimidester Funktion und eine quarternäre Ammoniumgruppe. Diese zwei Funktionalisierungen sind durch eine Gruppe R₁ getrennt. R₁ dient der Massenvariation der charge tags (die Kopplung der charge tags wird bei leicht basischem pH (8.5) in wäßriger Lösung auf Eis durchgeführt. Die Reaktion läuft in 30 Minuten vollständig ab).
Figur 3
   Prinzip der Synthese von PNA-Bibliotheken unter Verwendung parallel durchgeführter massengelabelter Synthesen sowie massengelabeiten Bausteinen L(randomisierte Position). In den randomisierten Positionen werden verschiedene Basen mit jeweils verschiedenen Substituenten am PNA-Rückgrat (als Massentags) verwendet. Die Masse des jeweiligen PNA-Moleküls ist eindeutig dessen Sequenz zugeordnet.
Figur 4
   Berechnetes Massenspektrum einer PNA-Bibliothek.
   Zwei verschiedene Festphasensynthesen (eine davon massengelabelt) mit je 32 verschiedenen Sequenzen ergeben 64 verschiedene Massenpeaks, von denen jeder zu einer spezifische Sequenz aus einer PNA-Bibliothek mit 3 variablen Positionen mit vier Basen (jeweils A, C, G und T eingesetzt) zugeordnet ist. Der Rechnung liegen die in Tabelle 1 aufgeführten Substituenten zugrunde. Für die Berechnung wurde das Computerprogramm MASP (© Dr. Christoph Steinbeck) verwendet.
Figur 5
   Mögliche PNA-Synthesebausteine für die kombinatorische Boc-Festphasensynthese, wie sie auch für die Synthese massenunterscheidbarer PNA-Bibliotheken verwendet werden. Es sind die in Tabelle 1 aufgeführten massengelabelten Bausteine dargestellt.
Figur 6
   Sequenzanalyse von Sonden durch spezifische Bindungsbrüche.
   Die verschiedenen Sequenzen einer Bibliothek von DNA-Sonden können massenspektrometrisch auch durch spezifische Bindungbrüche an den randomisierten Positionen (N = A, G, C oder T) identifiziert werden. Bereits während der Festphasensynthese wird an diesen Positionen eine Phosphorothioatfunktion eingeführt, an der die DNA spezifisch gespalten werden kann (z.B. mit lodoethanol). Dies ist schematisch, ohne Berücksichtigung der genauen chemischen Beschaffenheit der Fragmente, dargestellt. Die Masse der Fragmente erlaubt, da die Sequenz zum großen Teil bekannt ist, die eindeutige Bestimmung der Gesamtsequenz, ohne daß eine vollständige Sequenzierung erfolgen müßte. Der Substituent R1 bezeichnet eine beliebige weitere DNA-Sequenz, R2 eine weitere beliebige Sequenz oder -H. R3 bezeichnet -OH (Phosphodiester) oder Alkyl- (Alkylphosphonat).

### Die Beispiele erläutern die Erfindung.

### Beispiel 1: Darstellung einer Ausführungsform des gesamten erfinderischen Verfahrens

Eine Ziel-DNA wird mit einer Funktion hergestellt, die deren Immobilisierung an einer Oberfläche erlaubt. Die Ziel-DNA wird mittels PCR hergestellt und in dieser PCR ein mit Biotin funktionalisierter Primer verwendet. Für die Immobilisierung ist die Oberfläche streptavidinbeschichtet. Eine Bibliothek von massenunterscheidbaren Sonden, die in vorstehend beschriebener Weise hergestellt wurde, wird an die Ziel-DNA hybridisiert. Komplementäre Bestandteile der Sondenbibliothek binden an die entsprechenden Ziel-DNAs. Anschließend wird gründlich gewaschen, um nicht spezifisch gebundene Sonden zu entfernen. Die spezifischen Sonden werden durch Hitzedenaturierung von der Ziel-DNA gelöst. Die Lösung wird in einem Electrospray Massenspektrometer analysiert. Die gefundenen Massen und die Massenunterscheidbarkeit der Sonden lassen Rückschlüsse auf Teilsequenzen in der Ziel-DNA zu

### Beispiel 2: Analyse von Ziel-DNA, die über eine Biotin-Strepavidinbrücke immobilisiert ist

Eine Ziel-DNA wird durch eine Biotinfunktion an ein streptavidinbeschichtetes magnetic bead gebunden. Eine Bibliothek von massenunterscheidbaren Sonden wird an die Ziel-DNA hybridisiert. Unspezifische Hybridisierungen werden abgewaschen. Die spezifischen hybridisierten Sonden werden durch Erwärmen in wäßrige Lösung abgelöst. Die wäßrige Lösung wird in einem Electrospray Massenspektrometer analysiert. Die gefundenen Massen und die Massenunterscheidbarkeit der Sonden lassen Rückschlüsse auf Teilsequenzen in der Ziel-DNA zu.

### Beispiel 3: Immobilisierung von Ziel-DNA

In einer bevorzugten Variante des Verfahrens wird die Ziel-DNA an ein bead bestehend aus einem Epoxy-funktionalisierten Acrylpolymer gebunden. Die nicht umgesetzten Epoxyfunktionen werden nachfolgend mit einem Überschuß eines Amins desaktiviert. Weiter wird analog Beispiel 2 verfahren.

### Beispiel 4: Analyse verschiedener Ziel-DNAs in einer Mikrotiterplatte

In einer mit Streptavidin beschichteten Mikrotiterplatte werden 96 bzw. 384 verschiedene, biotinfunktionalisierte Ziel-DNAs je in einer Vertiefung immobilisiert. In jeder Vertiefung wird mit einer Bibliothek von massenunterscheidbaren Sonden hybridisiert. Die Platte wird gewaschen, um unspezifische Hybridisierungen zu lösen. Die spezifischen Hybridisierungen werden durch Erwärmen in wäßriger Lösung jeweils in der entsprechenden Vertiefung gelöst. Die wäßrige Lösung in jeder Vertiefung wird in einem Electrospray Massenspektrometer analysiert. Die gefundenen Massen und die Massenunterscheidbarkeit der Sonden lassen Rückschlüsse auf Teilsequenzen der Ziel-DNA in der entsprechenden Vertiefung zu.

### Beispiel 5: Synthese einer PNA-Bibliothek mit 64 verschiedenen Sequenzen

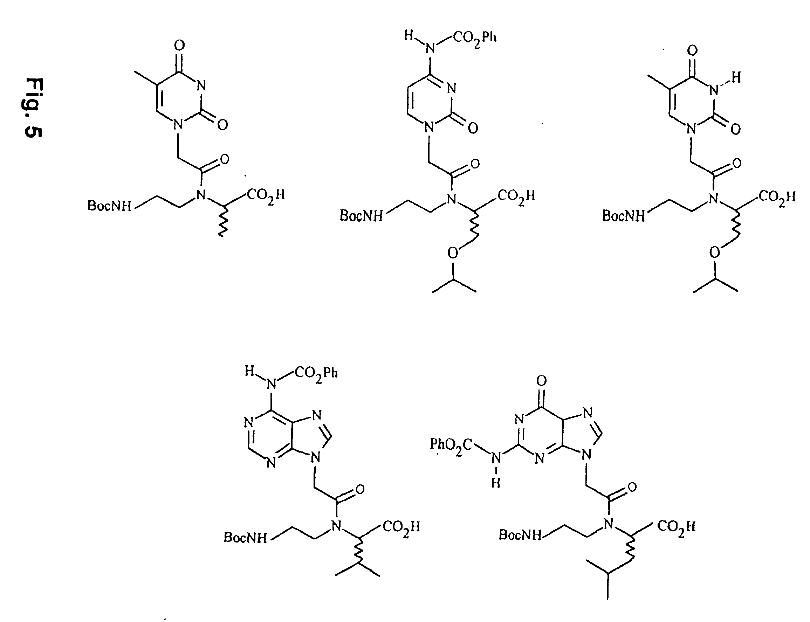

Ein Beispiel für eine PNA-Bibliothek mit eindeutiger Beziehung der Masse zur Sequenz zeigen Figur 4 und Tabelle 1. In einer sonst beliebigen PNA sollen drei Positionen in der kombinatorischen Festphasensynthese variiert werden. Dies entspricht bei 4 verschiedenen Basen 64 möglichen Verbindungen. Im vorliegenden Beispiel werden zwei getrennte Synthesen mit den in Tabelle 1 gegebenen Bausteinen durchgeführt. Synthese 2 wird zusätzlich durch das Anbringen von zwei Valin-Einheiten massengelabelt. Jede Teilsynthese liefert 32 Verbindungen, die alle über ihre Masse unterscheidbar sind. Beide Teilbibliotheken können nun zur Bibliothek mit 64 verschiedenen Sequenzen mit jeweils spezifischer Masse vereinigt werden.
Die 64 Peaks überlappen nicht, jeder entspricht einer spezifischen Sequenz aus einer PNA-Bibliothek mit drei randomisierten Basenpositionen (je 4 Basen).

### Beispiel 6: Aufbau einer Sondenbibliothek mit 448 Sonden

7 Synthesen werden nach der im letzten Beispiel beschriebenen Methode durchgeführt und anschließend die Massen/charge tags gekoppelt.

| | Massenbereich |
|---|---|
| 1. 64 Sonden: charge tag-TCP₁GAP₂GAP₃G | 2600-2800 Da |
| 2. 64 Sonden: charge tag+200 Da mass tag- TCP₁AGP₂GAP₃G | 2800-3000 Da |
| 3. 64 Sonden: charge tag+400 Da mass tag- TCP₁AGP₂AGP₃G | 3000-3200 Da |
| 4. 64 Sonden: charge tag+600 Da mass tag- TCP₁AAP₂AGP₃G | 3200-3400 Da |
| 5. 64 Sonden: charge tag+800 Da mass tag- TCP₁AAP₂GAP₃G | 3400-3600 Da |
| 6. 64 Sonden: charge tag+1000 Da mass tag- TCP₁GAP₂GAP₃G | 3600-3800 Da |
| 7. 64 Sonden: charge tag+1200 Da mass tag- TCP₁GAP₂AGP₃G | 3800-4000 Da |

In der vorgenannten Synthese-Serie stellt die sechste Synthese eine interne Kontrolle dar. Alternativ kann auch folgende Synthese durchgeführt werden:

6.64 Sonden: charge tag+ 1000 Da mass tag- TCP₁GGP₂GAP₃G 3600-3800 Da.

### Beispiel 7: Beschichtung einer Mikrotiterplatte mit Protein

Die Oberfläche einer Mikrotiterplatte wird mit Gene32, einem Protein das sequenzunspezifisch einzelsträngige DNA bindet, beschichtet. Nach der Beschichtung des Targets mit diesem Protein kann ein Array von Ziel-DNAs daraufgelegt werden. Wenn das Array der Ziel-DNAs aus cDNA besteht, sind diese in der PCR mit oligo-dT (z.B. dTTTTTTTTTTTTT) geprimt worden. Oligo-dT wechselwirkt stark mit Gene32. Die kovalente Bindung des oligo-dT zum gene32 kann durch ein Photocrosslinking mit kurzem UV-Licht erreicht werden.^{29,30} Nach dieser Immobilisierung kann eine Bibliothek von Sonden in der beschriebenen Art zur Analyse der Ziel-DNAs verwendet werden. Es bieten sich auch sequenzspezifische Protein/DNA-Wechselwirkungen an, wie z.B. GCN4/AP1.

### Beispiel 8: Immobilisierung von Ziel-DNA and einer Epoxyfunktionalisierten Festphase

10 mg Support (Eupergit C250 L, Röhm Pharma Polymere) werden in 1 ml Immobilisierungspuffer (1M K₂HPO₄/KH₂PO₄ pH 7,5) suspendiert. 1,5 nmol der zu immobilisierenden Ziel-DNA werden zugesetzt und 24 h bei Raumtemperatur unter sanftem Schütteln inkubiert. Der Überstand wird abgenommen und die nicht umgesetzten Epoxyfunktionen durch Behandlung mit 1 M Glycinlösung bei Raumtemperatur 24 h desaktiviert. Der Überstand wird abgenommen, der Support mehrfach gewaschen. Die immobilisierte DNA kann so bei -20 °C über längere Zeit aufbewahrt werden.

### Beispiel 9: Selektive Hybridisierung mit "charge tagged" PNAs

Die immobilisierte Ziel-DNA wird mit der Bibliothek von "charge tagged" PNA-Sonden (80 pmol in 20 µl/8 pmol immobilisierter DNA) in Hybridisationspuffer (10 mM Tris/HCl), 5 mM NH₄Cl, 15 % Formamid) 15 min. bei 65°C inkubiert und anschließend die nicht hybridisierten Sonden durch dreimaliges Waschen mit Hybridisationspuffer entfernt. Die Dehybridisierung erfolgt anschließend in 40 %igem Acetonitril bei 80 °C. Ein Aliquot dieser Lösung wird lyophilisiert, in Wasser aufgenommen und im ESI-Massenspektrometer analysiert.

**Tabelle 1:**

| An den PNA-Untereinheiten angebrachte Substituenten zur Herstellung einer massengelabelten Bibliothek mit eindeutiger Massen/Sequenz Beziehung. | | | |
|---|---|---|---|
| Base | Position 1 | Position 2 | Position 3 |
| A | H | *i*Pr | H |
| T | H | Me | *i*PrOCH₂ |
| C | H | H | *i*PrOCH₂* |
| G | H | *i*Bu | H * |

| | | | |
|---|---|---|---|
| * : Zweite Synthese, mit 2 Valin-Einheiten massengelabelt. Die entsprechenden Synthesebausteine zeigt Figur 5. | | | |

### Literatur

¹ Hoheisel, J.D. and Lehrach, H. 1993. Use of reference libraries and hybridsationfingerprint for relational genome analysis. FEBS. 325: 118-122.
² Scholler, P., Karger, A.E., Meier-Ewert, S., Lehrach, H., Delius, H. and Hoheisel, J.D. 1995. Fine-mapping of shotgun template-libraries; an efficient strategy for the systematic sequencing of genomic DNA. Nucleic Acids Res. 23: 3842-3849.
³ Brenner, S. 1994. Methods for sorting polynucleotides using oligonucleotide tags. US Patent. 5,604,097.
⁴ Brenner, S. 1995. Minimally cross-hybridizing sets of oligonucleotide tags. US Patent. 5,635,400.
⁵ Guo, Z., Guilfoyle, R.A., Thiel. A.J., Wang, R. and Smith, L.M. 1994. Direct fluorescence analysis of genetic polymorphisms by hybridization with oligonucleotides arrays of glass supports. Nucleic Acids Res. 22: 5456-5465.
⁶ Church, G.M. and Kieffer-Higgins, S. 1988. Multiplex DNA sequencing. Science. 240: 185-188.
⁷ Church, G.M. and Kieffer-Higgins, S. 1990. Multiplex analysis of DNA. US Patent. 5,149,625.
⁸ Arlinghaus, H.F., Kwoka, M.N., Guo, X.-Q. and Jacobson, K.B. 1997. Multiplex DNA sequencing and diagnostics by hybridization with enriched stable isotope labels. Anal. Chem. 69: 1510-1517.
⁹ Nielsen, P.E., Buchardt, O., Egholm, M. and Berg, R.H. 1993. Peptide nucleic acids. US Patent. 5,539,082.
¹⁰ Buchardt, O., Egholm, M., Berg, R.H. and Nielsen, P.E. 1993. Peptide nucleic acids and their potential applications in biotechnology. Trends in Biotechnology, 11: 384-386.
¹¹ Gut, I.G. and Beck, S. 1995. Method of nucleic acid analysis. Patent WO96/27681.
¹² Gut, I.G., Jeffery, W.A., Pappin, D.J.C. and Beck, S. 1997. Analysis of DNA by 'charge tagging' and matrix-assisted laser desorption/ionization mass spectrometry. Rapid Commun. Mass Spectrom. 11: 43-50.
¹³ Lowe, G. 1995. Combinatorial Chemistry. Chem. Soc. Rev. 24: 309.
¹⁴ Jung, G., Früchtel, J.S. 1996. Organische Chemie an fester Phase. Angew. Chem. 108: 19-46.
¹⁵ Choong, I.C., Ellman, J.A. 1996. Solid-Phase Synthesis: Applications to Combinatorial Libraries. Ann. Reports in Med. Chem. 31: 309.
¹⁶ Pirrung, M.C. 1997. Spatially Adressable Combinatorial Libraries. Chem. Rev. 97: 473.
¹⁷ Cook, P.D., Kiely, J. and Sprankle, K. 1994. Peptide nucleic acid combinatorial libraries and improved methods of synthesis. US Patent. 5,539,083.
¹⁸ Nielsen, P.E. Peptide nucleic acids: a new dimension to peptide libraries and aptamers. Methods in Enzymology. 426-433.
¹⁹ Metzger, J.W., Stevanovic, S., Brünjes, J., Wiesmüller, K.-H., Jung, G. 1994. Electrospray Mass Spectrometry amd Multiple Sequence Analysis of Synthetic Peptide Libraries. Methods: A Companion to Methods in Enzymology 6: 425-431.
²⁰ Loo, J.A., DeJohn, D.E., Andrews, P.C. 1996. Application of Mass Spectrometry for Characterizing and Identifying Ligands from Combinatorial Libraries. Ann. Reports in Med. Chem. 31:319.
²¹ Pomerantz, S.C., McCloskey, J.A., Eaton, B.C., 1997. Deconvolution of Combinatorial Oligonucleotide Libraries by Electrospray lonization Tandem Mass Spectrometry. J. Am. Chem. Soc. 119: 3861.
²² Carr, S.A., Benkovic, S.J., Winograd, N. 1996. Evaluation of Mass Spectrometric Methods Applicable to the Direct Analysis of Non-Peptide Bead-Bound Combinatorial Libraries. Anal. Chem. 68: 237.
²³ Uhlen, M. et al. 1988, Nucleic Acids Res. 16, 3025-3038.
²⁴ Chrisey, L.A., Lee, G.U. and O'Ferrall, C.E. 1996. Covalent attachment of synthetic DNA to self-assembled monolayer films. Nucleic Acids Res. 24: 3031-3039.
²⁵ Timofeev, E.N., Kochetkova, S.V., Mirzabekov, A.D. und Florentiev, V.L. 1996. Regioselective immobilization of short oligonucleotides to acrylic copolymer gels. Nucleic Acids Res. 24: 3142-3148.
²⁶ O'Donnell, M.J., Tang, K., Köster, H., Smith, C.L. and Cantor, C.R. 1997. Highdensity, covalent attachment of DNA to silicone wafers for analysis by MALDI-TOF mass spectrometry. Anal. Chem. 69: 2438-2443.
²⁷ Cantor, C.R. 1995. Methods of preparing probe array by hybridization. US Patent. 5,631,134.
²⁸ Bruick, R.K., Koppitz, M., Joyce, G.F. and Orgel, L.E. 1997. A simple procedure for constructing 5'-amino-terminated oligodeoxynucleotides in aqueous solution. Nucleic Acids Res. 25: 1309-1310.
²⁹ Hockensmith, J.W., Kubasek, W.L., Vorachek, W.R. and von Hippel, P.H. 1993. Laser cross-linking of proteins to nucleic acids. J. Bio. Chem. 268: 15712-15720.
³⁰ von Hippel, P.H. 1994. Protein-DNA recognition: New perspectives and underying themes. Science. 263: 769-770.

## Patentansprüche

1. Verfahren zum Nachweis einer Nucleotidsequenz in einem Nucleinsäuremolekül, das die folgenden Schritte umfaßt:
(a) Hybridisierung von Nucleinsäuremolekülen mit einem Satz von Sonden unterschiedlicher Nucleobasensequenzen, wobei jede Sonde eine von allen anderen Sonden verschiedene Masse aufweist;
(b) Abtrennung der nicht hybridisierten Sonden;
(c) Ablösen der spezifisch hybridisierten Sonden in ein Lösungsmittel;
(d) Analyse der hybridisierten Sonden in der Lösung mittels Electrospray Massenspektrometrie; und
(e) Bestimmung der Nucleinsäuremoleküle durch die daran hybridisierten Sonden.

2. Verfahren nach Anspruch 1, wobei die Nucleinsäuremoleküle vor oder nach Schritt (a) an der Oberfläche eines Trägers immobilisiert werden.

3. Verfahren nach Anspruch 2, wobei die Immobilisierung der Nucleinsäuremoleküle an der Oberfläche über eine NH₂-, Epoxy- oder SH-Funktion, vermittels der Beschichtung der Probenträgeroberfläche mit einem Silikat oder Silan, über eine Protein-Substrat-, Protein-Protein- oder eine Protein-Nucleinsäure-Interaktion, oder über eine Wechselwirkung zweier hydrophober Komponenten erfolgt.

4. Verfahren nach Anspruch 3, wobei die Protein-Substrat-Interaktion eine Biotin-Streptavidinbindung oder eine Antikörper-Antigenbindung ist.

5. Verfahren nach Anspruch 3, wobei die Protein-Nucleinsäure-Interaktion eine Gene32-Nucleinsäurebindung ist.

6. Verfahren nach einem der Ansprüche 1 bis 5, wobei die Sonden Nucleinsäuren sind, die mit einem Massen-Tag versehen sind.

7. Verfahren nach Anspruch 6, wobei der Massen-Tag zugleich Ladungs-Tag ist.

8. Verfahren nach Anspruch 6, wobei die Nucleinsäuren zusätzlich mit einem Ladungs-Tag versehen sind.

9. Verfahren nach einem der Ansprüche 1 bis 8, wobei die Sonden PNAs, alkylierte Phosphorothioatnucleinsäuren oder Alkylphosphonatnucleinsäuren sind.

10. Verfahren nach einem der Ansprüche 1 bis 9, wobei die Sonden durch kombinatorische Festphasen-Synthese hergestellt werden.

11. Verfahren nach Anspruch 10, wobei unterschiedliche Basenbausteine derart markiert sind, daß die aus ihnen synthetisierten Sonden jeweils über ihre Massen im Massenspektrometer unterscheidbar sind.

12. Verfahren nach Anspruch 11, wobei die Markierung eine Methyl-, Ethyl-, Propyl-, eine verzweigte oder unverzweigte Alkyl-, eine halogensubstituierte verzweigte oder unverzweigte Alkyl-, Alkoxyalkyl-, Alkylaryl-, Arylalkyl-, Alkoxyaryl- oder Aryloxyalkyl-Gruppe ist oder eine von deren deuterierten oder sonstigen isotopen Varianten.

13. Verfahren nach einem der Ansprüche 9 bis 12, wobei die Sonden mit mindestens einer Modifikation in einer definierten Position von randomisierten Nucleotiden entfernt versehen sind, die eine Spaltung der Sonde erlaubt.

14. Verfahren nach Anspruch 13, wobei die Modifikation die Einführung einer Phosphorothioatgruppe und/oder einer RNA-Base und/oder einer Phosphotriesterbindung in die Sonde ist.

15. Verfahren nach einem der Ansprüche 1 bis 14, wobei die Sonden als Teilbibliotheken hergestellt werden, die mit unterschiedlichen Massenund/oder Ladungs-Tags versehen sind.

16. Verfahren nach einem der Ansprüche 1 bis 15, wobei die Positionen der Sonden auf dem Probenträger eine Zuordnung zu dem damit hybridisierenden Nucleinsäuremolekül erlaubt.

## Claims

1. A method for detecting a nucleotide sequence in a nucleic acid molecule comprising the following steps:
(a) hybridization of nucleic acid molecules to a set of probes of different nucleobase sequences wherein each probe has a mass that differs from the one of all the other probes;
(b) separation of the probes that were not hybridized;
(c) detachment of the specifically hybridized probes into a solvent;
(d) analysis of the hybridized probes in a solution by means of electrospray mass spectrometry; and
(e) determination of the nucleic acid molecules by means of the probes hybridized to them.

2. The method according to claim 1 wherein the nucleic acid molecules are immobilized at the surface of a support before or after step (a).

3. The method according to claim 2 wherein the immobilization of the nucleic acid molecules at the surface is carried out via an NH₂, epoxy or SH function by means of coating the surface of the probe supports with a silicate or silane, via a protein-substrate, protein-protein or a protein-nucleic acid interaction or via an interaction of two hydrophobic components.

4. The method according to claim 3 wherein the protein-substrate interaction is a biotin-streptavidin binding or an antibody-antigen binding.

5. The method according to claim 3 wherein the protein-nucleic acid interaction is a Gene32-nucleic acid binding.

6. The method according to any one of claims 1 to 5 wherein the probes are nucleic acids having a mass tag.

7. The method according to claim 6 wherein the mass tag is at the same time a charge tag.

8. The method according to claim 6 wherein the nucleic acids additionally have a charge tag.

9. The method according to any one of claims 1 to 8 wherein the probes are PNAs, alkylated phosphorothioate nucleic acids or alkylphosphonate nucleic acids.

10. The method according to any one of claims 1 to 9 wherein the probes are generated by means of combinatorial solid phase synthesis.

11. The method according to claim 10 wherein different base building blocks are labelled in such a way that the probes synthesized therefrom can be differentiated in the mass spectrometer due to their mass.

12. The method according to claim 11 wherein the labelling is a methyl, ethyl, propyl, a branched or non-branched alkyl, a halogen substituted branched or non-branched alkyl, alkoxyalkyl, alkylaryl, arylalkyl, alkoxyaryl or aryloxyalkyl group or one of their deuterated or other isotopic variants.

13. The method according to any one of claims 9 to 12 wherein the probes have at least one modification in a defined position away from randomized nucleotides allowing for the cleavage of the probe.

14. The method according to claim 13 wherein modification means the introduction of a phosphorothioate group and/or an RNA base and/or a phosphotriester bond into the probe.

15. The method according to any one of claims 1 to 14 wherein the probes are generated as partial libraries having different mass and/or charge tags.

16. The method according to any one of claims 1 to 15 wherein the positions of the probes on the probe support allow for an allocation to the nucleic acid molecule hybridizing thereto.

## Revendications

1. Procédé de détection d'une séquence nucléotidique dans une molécule d'acide nucléique, qui comprend les étapes suivantes :
(a) hybridation d'une molécule d'acide nucléique avec une série de sondes de séquences de bases nucléotidiques différentes, chaque sonde présentant une masse distincte de toutes les autres ;
(b) élimination des sondes non hybridées ;
(c) dissolution des sondes spécifiquement hybridées dans un agent de solubilisation,
(d) analyse des sondes hybridées dans la solution par spectrométrie de masse electrospray ; et
(e) identification de la molécule d'acide nucléique par le biais de la sonde hybridée.

2. Procédé selon la revendication 1, dans laquelle les molécules d'acide nucléique sont immobilisées à la surface d'un support avant ou après l'étape (a).

3. Procédé selon la revendication 2, dans laquelle l'immobilisation des molécules d'acide nucléique est effectuée en surface par une fonction NH₂-, époxy ou SH-, au moyen du revêtement de la surface du support de l'essai avec un silicate ou un silane, par une interaction protéine-substrat, protéine-protéine ou protéine-acide nucléique, ou par l'effet d'échange de deux composants hydrophobes.

4. Procédé selon la revendication 3, dans laquelle l'interaction protéine-substrat est une liaison biotine-streptavidine ou anticorps-antigène.

5. Procédé selon la revendication 3, dans laquelle l'interaction protéine-acide nucléique est une liaison gène 32-acide nucléique

6. Procédé selon l'une des revendications 1 à 5, dans laquelle les sondes sont des acides nucléiques pourvus d'une étiquette de masse.

7. Procédé selon la revendication 6, dans laquelle les étiquettes de masse sont simultanément des étiquettes de charge.

8. Procédé selon la revendication 6, dans laquelle les acides nucléiques sont en plus pourvus d'une étiquette de charge.

9. Procédé selon l'une des revendications 1 à 8, dans laquelle les sondes sont des PNA, des phosphorothio-acides nucléiques alkylés, ou des alkylphosphonato-acides nulcéiques.

10. Procédé selon l'une des revendications 1 à 9, dans laquelle les sondes sont produites par synthèse combinatoire en phase solide.

11. Procédé selon la revendication 10, dans laquelle des motifs unitaires des bases sont marqués de sorte que les sondes synthétiques qu'ils composent se distinguent respectivement par leur masse dans le spectromètre de masse.

12. Procédé selon la revendication 11, dans laquelle le marqueur est un groupe méthyle, éthyle, propyle, alkyle ramifié ou non ou un groupe alkyle, alkoxyalkyle, alkylaryle, arylalkyle, alkoxyaryle ou aryloxyalkyle ramifié ou non, substitué par un halogène, ou une de leurs variantes deutériées ou une autre variante isotopique.

13. Procédé selon l'une des revendications 9 à 12, dans laquelle les sondes sont prévues avec au moins une modification dans une position définie éloignée de nucléotides randomisés, qui permette un clivage de la sonde.

14. Procédé selon la revendication 13, dans laquelle la modification consiste en une insertion d'un groupe phosphorothio et/ou d'une base d'ARN et/ou d'une liaison phosphotriester dans la sonde.

15. Procédé selon l'une des revendications 1 à 14, dans laquelle les sondes sont préparées en tant que banques partielles, qui sont pourvues d'étiquettes de masse et/ou de charge différentes.

16. Procédé selon l'une des revendications 1 à 15, dans laquelle les positions des sondes sur le support d'essai permettent un classement dans la molécule d'acide nucléique qui hybride.
